(19) Europäisches Patentamt / European Patent Office / Office européen des brevets

(11) **EP 4 268 730 A1**

(12) **EUROPEAN PATENT APPLICATION**
published in accordance with Art. 153(4) EPC

(43) Date of publication:
**01.11.2023 Bulletin 2023/44**

(21) Application number: **21910801.6**

(22) Date of filing: **21.12.2021**

(51) International Patent Classification (IPC):
**A61B 10/00** *(2006.01)*    **A61B 5/02** *(2006.01)*

(52) Cooperative Patent Classification (CPC):
**A61B 5/02; A61B 10/00**

(86) International application number:
**PCT/JP2021/047384**

(87) International publication number:
**WO 2022/138661 (30.06.2022 Gazette 2022/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.12.2020 JP 2020216927**

(71) Applicant: **Astellas Pharma Inc.**
**Chuo-ku**
**Tokyo 103-8411 (JP)**

(72) Inventors:
• **OGINO, Makoto**
**Tokyo 103-8411 (JP)**
• **KAMADA, Hiroyuki**
**Morioka-shi, Iwate 020-0021 (JP)**

(74) Representative: **V.O.**
**P.O. Box 87930**
**2508 DH Den Haag (NL)**

(54) **ARRHYTHMIC STATE DETECTION ASSISTING DEVICE AND PROGRAM**

(57) An arrhythmic state detection assisting device includes an acquisition unit, a derivation unit and an estimation unit. The acquisition unit acquires image information obtained by capturing an image of a predetermined part of a subject with an image capture device that captures a color moving image. The derivation unit derives a variance value, for a predetermined interval, of periods of a periodic change in a green luminance component in the image information acquired by the acquisition unit. As the variance value derived by the derivation unit becomes greater, the estimation unit estimates a higher likelihood that an arrhythmia is occurring in the subject.

FIG.2

EP 4 268 730 A1

**Description**

TECHNICAL FIELD

The present disclosure relates to an arrhythmic state detection assisting device and a program.

BACKGROUND ART

Heretofore, the following technologies have been applicable to using the pulse of a user to assist in health management of the user.

Japanese Patent Application Laid-Open (JP-A) No. 2019-58258 discloses a physical and mental state assessment device with the object of providing a technology that accurately assesses a physical or mental state, such as stress or the like, of a test subject without imposing a burden on the test subject. The physical and mental state assessment device includes calculating means for calculating a first similarity representing a degree of similarity between a first pulse rate in a first interval and a second pulse rate in the first interval, for a first region including a part of the face in a time series of plural face images. The first pulse rate is estimated by a first estimation method using plural luminance values of the first region, and the second pulse rate is estimated by a second estimation method that is different from the first estimation method. The physical and mental state assessment device also includes assessing means for, in accordance with the calculated first similarity, using any of the first pulse rate, the second pulse rate and the plural face images in the first interval to assess the physical or mental state of the test subject.

JP-A No. 2019-136352 discloses a bio-information display device with the object of improving ease of use when heartbeat fluctuation biofeedback is conducted. The bio-information display device includes a casing and an imaging unit, which captures images of the face of an observation subject and acquires video image data of a detection region from the captured images. The detection region includes a skin region of the face of the observation subject. The bio-information display device further includes a processing unit that acquires bio-information including information on pulse waves of the person from the video image data of the detection region and that uses this bio-information to generate an information display screen for implementing heartbeat fluctuation biofeedback. The bio-information display device includes a display unit that is disposed at the casing, in the same plane as the imaging unit, and that displays the information display screen. The processing unit of the bio-information display device generates the information display screen to include a breathing assistant that aids breathing of the observation subject for the heartbeat fluctuation biofeedback. In the information display screen of the bio-information display device, at least a portion of the breathing assistant is disposed on a straight line passing through the imaging unit. The straight line passing through the imaging unit orthogonally crosses an outer periphery edge of the display unit that is closest to the imaging unit.

Japanese Patent Application Re-publication No. 2017-85894 discloses a pulse wave analysis device with the object of enabling an improvement in analytical accuracy of pulse wave waveforms of a subject. The pulse wave analysis device extracts images of each of plural parts of a subject from plural captured images in which the subject is imaged, and generates pulse wave waveforms of the respective parts by analyzing the images of the respective parts extracted from the plural captured images. The pulse wave analysis device then calculates a first matching rate representing a degree of matching between pulse wave waveforms among the generated pulse wave waveforms of the respective parts.

U.S. Patent No. 10,004,410 discloses a technology that uses images of the face of a subject to detect information relating to pulse waves, including an arrhythmia of the subject. In this technology, red and green luminance components of the images of the face of the subject are used to estimate whether or not an arrhythmia is occurring in the subject.

SUMMARY OF INVENTION

TECHNICAL PROBLEM

As a result of investigations by the inventors of the present invention, it has been discovered that an occurrence of arrhythmia in a subject correlates strongly with a variance value, for a predetermined interval, of periods of a periodic change in a green luminance component in image information acquired by image capture of a predetermined part of the subj ect.

However, the technologies disclosed in the reference documents described above give no consideration to this variance value. When these technologies are employed, it is not necessarily possible to accurately assess whether or not an arrhythmia is occurring in a subj ect.

In consideration of the problem described above, an object of the present disclosure is to provide an arrhythmic state detection assisting device and program that may accurately estimate whether or not a likelihood that an arrhythmia is occurring is high.

SOLUTION TO PROBLEM

A first aspect of the present disclosure includes: an acquisition unit that acquires image information obtained by capturing an image of a predetermined part of a subject with an image capture device that captures a color moving image; a derivation unit that derives a variance value, for a predetermined interval, of periods of a periodic change in a green luminance component in the image information acquired by the acquisition unit; and an estimation unit that, as the variance value derived by the derivation unit becomes greater, estimates a higher likelihood that an arrhythmia is occurring in the subject.

In a second aspect of the present disclosure, in the first aspect, the estimation unit estimates a higher likelihood that an arrhythmia is occurring when the variance value is at least a predetermined threshold value.

In a third aspect of the present disclosure, in the first aspect or the second aspect, the part is a face of the subject.

In a fourth aspect of the present disclosure, in the third aspect, the part is parts at plural portions of the face.

In a fifth aspect of the present disclosure, in any one of the first to fourth aspects, when the part moves in the moving image represented by the image information acquired by the acquisition unit, the derivation unit tracks movement of the part for deriving the variance value.

In a sixth aspect of the present disclosure, any one of the first to fifth aspects further includes an identifying unit that, when the subject has an arrhythmia, identifies a type of the arrhythmia, the identifying unit using a chronological pattern of the green luminance component.

A seventh aspect of the present disclosure execute by a computer to perform processing including: acquiring image information obtained by capturing an image of a predetermined part of a subject with an image capture device that captures a color moving image; deriving a variance value, for a predetermined interval, of periods of a periodic change in a green luminance component in the acquired image information; and, as the derived variance value becomes greater, estimating a higher likelihood that an arrhythmia is occurring in the subject.

ADVANTAGEOUS EFFECTS OF INVENTION

According to the present disclosure, a likelihood that an arrhythmia is occurring may be more accurately estimated.

BRIEF DESCRIPTION OF DRAWINGS

Fig. 1 is a block diagram showing an example of hardware structures of an arrhythmic state detection assisting device according to an exemplary embodiment.
Fig. 2 is a block diagram showing an example of functional structures of the arrhythmic state detection assisting device according to the exemplary embodiment.
Fig. 3 is a schematic diagram showing an example of structures of a green luminance component information database according to the exemplary embodiment.
Fig. 4 is a schematic diagram showing an example of structures of an arrhythmia pattern information database according to the exemplary embodiment.
Fig. 5 is a diagram supporting a description of the theory of a technique for estimating a state of occurrence of arrhythmia according to the exemplary embodiment, which is a schematic diagram showing structures of an experiment by the inventors of the present invention.
Fig. 6 is a diagram supporting the description of the theory of the technique for estimating a state of occurrence of arrhythmia according to the exemplary embodiment, which is a graph showing states of variance values obtained in the experiment by the inventors of the present invention.
Fig. 7 is a diagram supporting the description of the theory of the technique for estimating a state of occurrence of arrhythmia according to the exemplary embodiment, which is a graph supporting a description of an instantaneous pulse rate employed in the experiment by the inventors of the present invention.
Fig. 8 is a flowchart showing an example of arrhythmic state detection assisting processing according to the exemplary embodiment.
Fig. 9 is an elevation view showing an example of composition of an arrhythmia presentation screen that is displayed at a time of execution of the arrhythmic state detection assisting processing according to the exemplary embodiment.
Fig. 10 is an elevation view showing an example of composition of a sinus rhythm presentation screen that is displayed at a time of execution of the arrhythmic state detection assisting processing according to the exemplary embodiment.

DETAILED DESCRIPTION

Below, an example of an exemplary embodiment of the present disclosure is described in detail with reference to the drawings. In the drawings, the same reference symbols are assigned to structural elements and portions that are the same or equivalent. Proportional dimensions in the drawings may be exaggerated to facilitate understanding and may be different from actual proportions.

In the present exemplary embodiment, a situation is described in which an arrhythmic state detection assisting device according to the technology of the present disclosure is applied at a general-purpose smartphone. However, application objects of the technology of the present disclosure are not limited to smartphones. As well as obvious alternative portable information processing devices such as portable video game machines, tablet terminals, notebook computers and the like, the technology may be applied to static information processing devices.

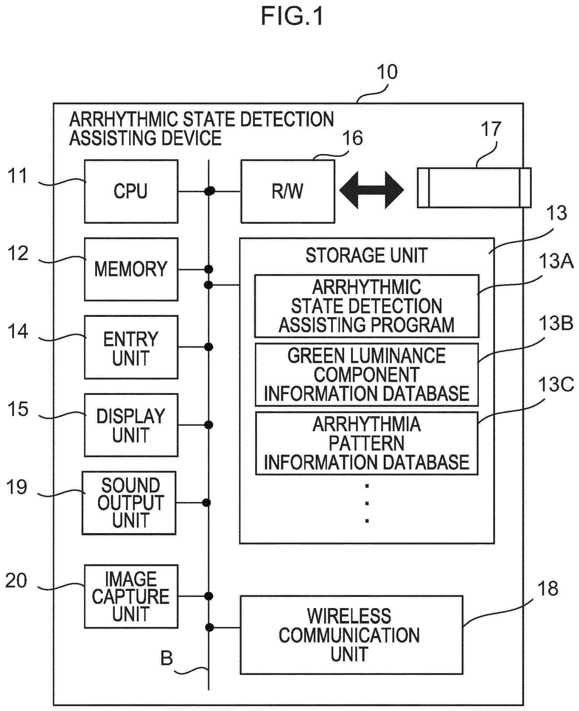

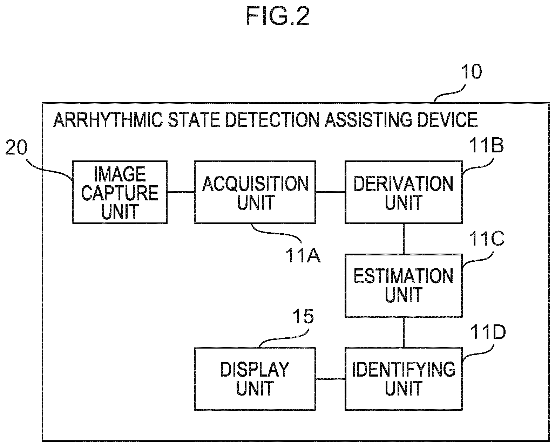

First, structures of an arrhythmic state detection assisting device 10 according to the present exemplary embodiment are described with reference to Fig. 1 and Fig. 2. Fig. 1 is a block diagram showing an example of hardware structures of the arrhythmic state detection assisting device 10 according to the exemplary embodiment. Fig. 2 is a block diagram showing an example of functional structures of the arrhythmic state detection assisting device 10 according to the exemplary embodiment.

As shown in Fig. 1, the arrhythmic state detection assisting device 10 according to the present exemplary embodiment is provided with a central processing unit (CPU) 11, a memory 12 that serves as a temporary memory region, a nonvolatile storage unit 13, and an entry unit 14 such as a touch panel with various switches or the like. The arrhythmic state detection assisting device 10 according to the present exemplary embodiment is further provided with a display unit 15 such as a liquid crystal display or the like and a medium reading/writing apparatus (R/W) 16. The arrhythmic state detection assisting device 10 according to the present exemplary embodiment is still further provided with a wireless communication unit 18 that conducts mobile communications by a predetermined communications standard, a sound output unit 19, and an image capture unit 20 that functions as an image capture device that captures color moving images. The CPU 11, the memory 12, the storage unit 13, the entry unit 14, the display unit 15, the medium reading/writing apparatus 16, the wireless communication unit 18, the sound output unit 19 and the image capture unit 20 are connected to one another via a bus B. The medium reading/writing apparatus 16 reads information that has been written to a recording medium 17 and writes information to the recording medium 17.

The storage unit 13 is realized by a hard disk drive (HDD), a solid state drive (SSD), flash memory or the like. The storage unit 13, serving as a storage medium, memorizes an arrhythmic state detection assisting program 13A. The arrhythmic state detection assisting program 13A is written to the recording medium 17, and the arrhythmic state detection assisting program 13A is memorized into the storage unit 13 by the recording medium 17 being set in the medium reading/writing apparatus 16 and the medium reading/writing apparatus 16 reading the arrhythmic state detection assisting program 13A from the recording medium 17. The CPU 11 loads the arrhythmic state detection assisting program 13A from the storage unit 13 into the memory 12, and sequentially executes processes of the arrhythmic state detection assisting program 13A.

Thus, in the arrhythmic state detection assisting device 10 according to the present exemplary embodiment, the arrhythmic state detection assisting program 13A is installed at the arrhythmic state detection assisting device 10 via the recording medium 17. However, this is not limiting. For example, a mode is possible in which the arrhythmic state detection assisting program 13A is installed at the arrhythmic state detection assisting device 10 by being downloaded via the wireless communication unit 18.

The storage unit 13 also memorizes a green luminance component information database 13B and an arrhythmia pattern information database 13C. The green luminance component information database 13B and arrhythmia pattern information database 13C are described in more detail below.

Now, functional structures of the arrhythmic state detection assisting device 10 according to the present exemplary embodiment are described with reference to Fig. 2. As shown in Fig. 2, the arrhythmic state detection assisting device 10 according to the present exemplary embodiment includes an acquisition unit 11A, a derivation unit 11B, an estimation unit 11C and an identifying unit 11D. The CPU 11 of the arrhythmic state detection assisting device 10, by executing the arrhythmic state detection assisting program 13A, functions as the acquisition unit 11A, the derivation unit 11B, the estimation unit 11C and the identifying unit 11D.

The acquisition unit 11A according to the present exemplary embodiment acquires image information that is obtained by capturing an image of a predetermined part of a subject with an image capture device that captures color moving images (the image capture unit 20 in the present exemplary embodiment).

In the present exemplary embodiment, the face of the subject is employed as the above-mentioned predetermined part, but this is not limiting. For example, any region may be employed as the predetermined part provided the skin of the region is exposed, such as a hand, arm, neck or the like of the subject. Furthermore in the present exemplary embodiment, parts at plural portions of the face of the subject are employed as the predetermined part. In the present exemplary embodiment, parts at two portions of the forehead and the left cheek are employed as the above-mentioned parts of plural portions of the face, but this is not limiting. For example, a mode is possible in which a combination of plural parts including, as well as these parts, other parts of the face of the subject such as the right cheek, the chin and the like is employed as the parts of plural portions of the face.

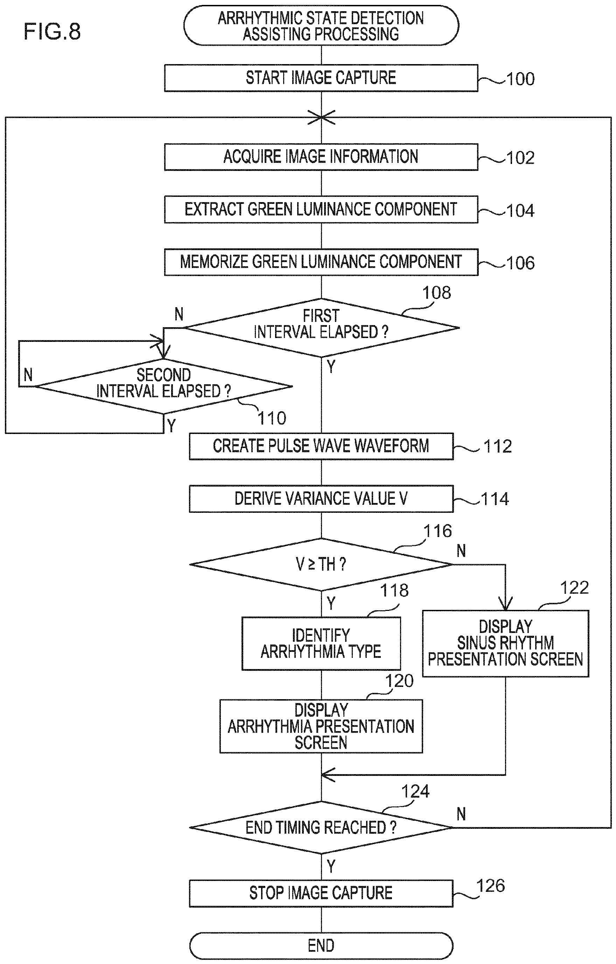

The derivation unit 11B according to the present exemplary embodiment derives a variance value, for a predetermined interval, of periods of a periodic change in a green luminance component in the image information acquired by the acquisition unit 11A (below referred to simply as "the variance value"). The estimation unit 11C according to the present exemplary embodiment estimates a higher likelihood that an arrhythmia is occurring in the subject as the variance value derived by the derivation unit 11B becomes greater. In particular, the estimation unit 11C according to the present exemplary embodiment estimates a higher likelihood that the subject is having an arrhythmia when the variance value is equal to or greater than a predetermined threshold value TH, which is described below (see Fig. 8).

In the present exemplary embodiment, as the threshold value TH, a value that is obtained by prior experimentation, a computer simulation or the like is employed as a value such that, when the variance value is equal to or greater than that value, a likelihood that an arrhythmia is occurring in the subject may be considered high. However, this mode is not limiting. For example, a mode is possible in which a threshold value TH is specified in advance for an individual subject in accordance with an arrhythmia detection accuracy required by the arrhythmic state detection assisting device 10, a state of movement of the subject, and the sex, age group and the like of the subject.

As described above, the present exemplary embodiment is a mode in which a high likelihood that an arrhythmia is occurring at the subject is estimated when the variance value derived by the derivation unit 11B is at least the predetermined threshold value TH, but this is not limiting. For example, a mode is possible in which the likelihood that an arrhythmia is occurring at the subject is estimated to be high when a difference between the variance value derived by the derivation unit 11B and a variance value of the individual subject in a previous predetermined interval is at least a predetermined threshold value. The variance value of the previous predetermined interval that is employed in this mode may be, for example, a mean value of the variance values in a recent predetermined interval (for example, 10 minutes), a mean value of the variance values in a predetermined interval (for example, five minutes) at the same time on a previous day, or the like.

When the part moves in the moving image represented by the image information acquired by the acquisition unit 11A, the derivation unit 11B according to the present exemplary embodiment tracks movement of the part to derive the variance value.

When the subject is having an arrhythmia, the identifying unit 11D according to the present exemplary embodiment uses a chronological pattern of the green luminance component mentioned above to identify a type of the arrhythmia.

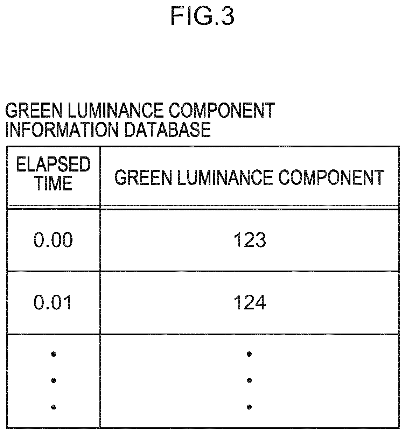

Now, the green luminance component information database 13B according to the present exemplary embodiment is described with reference to Fig. 3. Fig. 3 is a schematic diagram showing an example of structures of the green luminance component information database 13B according to the exemplary embodiment.

The green luminance component information database 13B according to the present exemplary embodiment is a database for memorizing information obtained by the image capture of moving images by the image capture unit 20 of the arrhythmic state detection assisting device 10, in order to detect when a likelihood that an arrhythmia is occurring in the subject is high.

As shown in Fig. 3, the green luminance component information database 13B according to the present exemplary embodiment memorizes information relating to green luminance components (below referred to as the green luminance component) at respective amounts of elapsed time with a predetermined interval (0.01 s in the present exemplary embodiment) from the start of image capture by the image capture unit 20.

The image information obtained by the image capture unit 20 includes separate luminance components of the primary colors red, green and blue, but the green luminance component information database 13B according to the present exemplary embodiment memorizes a chronological sequence only of information on the green luminance component of the separate luminance components of the primary colors. However, this is not limiting. A mode is possible in which information on the luminance components of all the colors red, green and blue is memorized.

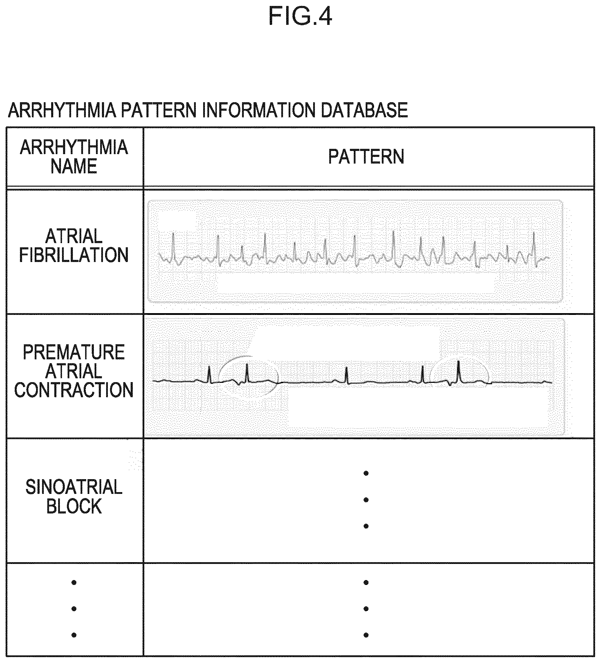

Now, the arrhythmia pattern information database 13C according to the present exemplary embodiment is described with reference to Fig. 4. Fig. 4 is a schematic diagram showing an example of structures of the arrhythmia pattern information database 13C according to the exemplary embodiment.

The arrhythmia pattern information database 13C according to the present exemplary embodiment is a database for identifying the type of arrhythmia when the likelihood that the subject is having an arrhythmia is high.

As shown in Fig. 4, the arrhythmia pattern information database 13C according to the present exemplary embodiment memorizes information on arrhythmia names and patterns for each of types of arrhythmia that are anticipated at the arrhythmic state detection assisting device 10 according to the present exemplary embodiment. These patterns are information representing chronological waveforms of pulse rates that are typical of the corresponding arrhythmias.

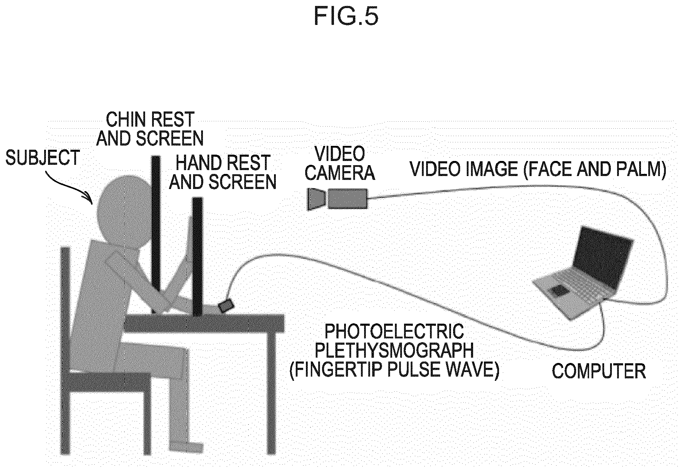

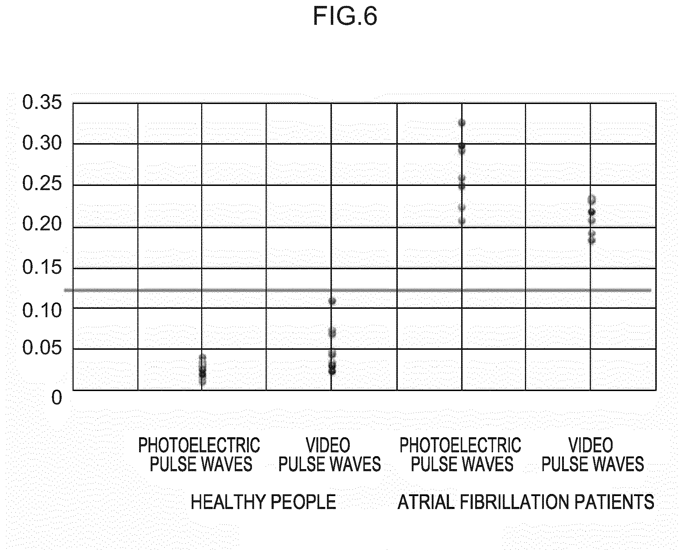

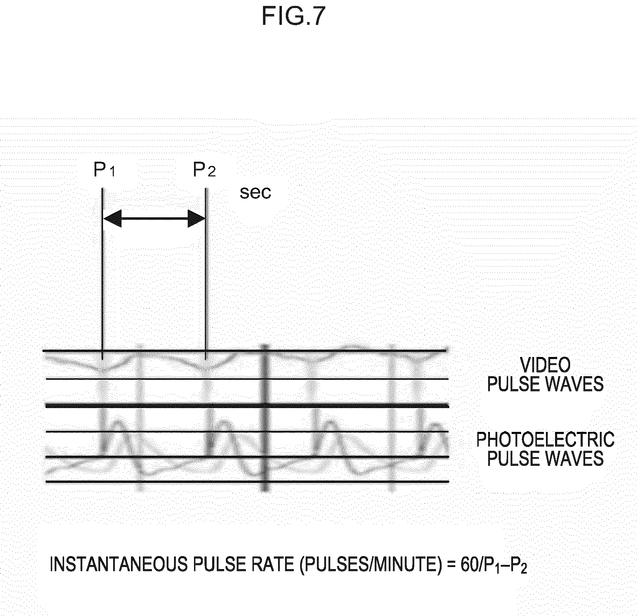

The theory of a technique for estimating a state of occurrence of arrhythmia in arrhythmic state detection assisting processing according to the present exemplary embodiment, which is described below, is now described with reference to Fig. 5 to Fig. 7. Fig. 5 is a diagram supporting the description of the theory of the technique for estimating a state of occurrence of arrhythmia according to the exemplary embodiment, which is a schematic diagram showing structures of an experiment by the inventors of the present invention. Fig. 6 is another diagram supporting the description of the theory of the technique for estimating a state of occurrence of arrhythmia according to the exemplary embodiment, which is a graph showing states of variance values obtained in the experiment by the inventors of the present invention. Fig. 7 is a further diagram supporting the description of the theory of the technique for estimating a state of occurrence of arrhythmia according to the exemplary embodiment, which is a graph supporting a description of an instantaneous pulse rate employed in the experiment by the inventors of the present invention.

It is known that analyzing body video images captured by an ordinary image capture device can provide, from green luminance components of the body video images, pulse wave signals (below referred to as video pulse waves) that are similar to pulse wave signals obtained from existing photoelectric plethysmographs. However, there are few examples of studies observing video pulse waves of patients with arrhythmias affecting the video pulse waves. Accordingly, the inventors of the present invention observed effects of arrhythmias on video pulse waves in, among arrhythmia patients, atrial fibrillation patients.

The subjects of these observations were 10 healthy volunteers, 2 men and 8 women, and 34 arrhythmia patients admitted to Iwate Medical University Hospital for ablation treatments (31 were sustained atrial fibrillation patients, 2 were patients who developed sinus rhythms while in hospital, and 1 was a patient with pacing by an internal pacemaker). Measurements were actually conducted on 50 atrial fibrillation patients and 12 healthy adults, but cases with very low confidence values due to the effects of environmental conditions during measurement and suchlike were excluded; so the healthy people and arrhythmia patients listed above were the subjects of observation.

As shown in Fig. 5, a test subject sits on a chair placed in front of a video camera for capturing moving images, and the head of the test subject is set in place by a support rest. The test subject wears dark glasses to protect their privacy, and images of the test subject's face are captured. A photoelectric plethysmograph is attached to a finger of the test subject.

In this state, a moving image of a region of the face of the test subject is captured while a photoelectric pulse wave and an electrocardiogram are measured for one minute, and the obtained moving image is analyzed to provide a video pulse wave. On the basis of the video pulse wave and the photoelectric pulse wave and electrocardiogram, comparative observations regarding pulse rates and pulse waves are conducted. In the example shown in Fig. 5, a structure in which a "hand rest and screen" are provided to enable imaging of a palm of the test subject is shown, but these measurements are excluded from consideration here.

The results were sinus rhythms with mean pulse rates y of video pulse waves standardized against mean pulse rates x of the photoelectric pulse waves. For healthy adults, y=1.0009x-0.0389 (correlation coefficient R=0.99) and, similarly for atrial fibrillation patients, the average pulse rate y=0.9832x (correlation coefficient R=0.8), confirming a correlation between the photoelectric pulse waves and video pulse waves.

As the variance value of periods of a periodic change in the video pulse rate for a predetermined interval, the inventors of the present invention employ a variance value V obtained from expression (1) below. This variance value V is obtained using an instantaneous pulse rate IP (pulses/minute) that is obtained by substituting a time difference (seconds) between a peak P1 and peak P2 of adjacent pulse waves at respective times, as in the example shown in Fig. 7, into expression (2).

$$V = \text{standard deviation of IP / mean pulse rate for 1 minute} \qquad (1)$$

in which

$$IP = 60 / P_1 - P_2 \qquad (2)$$

As shown in Fig. 6, the variance values V obtained by the observations described above from video pulse waves were $0.06 \pm 0.03$ for healthy people and $0.20 \pm 0.03$ for atrial fibrillation patients. Thus, it was confirmed that healthy people and atrial fibrillation patients may be substantially distinguished by the variance values V obtained from video pulse waves.

Given the results of the observations described above, the arrhythmic state detection assisting device 10 according to the present exemplary embodiment is a device that uses the variance value V to estimate whether or not a likelihood that an arrhythmia is occurring at a subject is high.

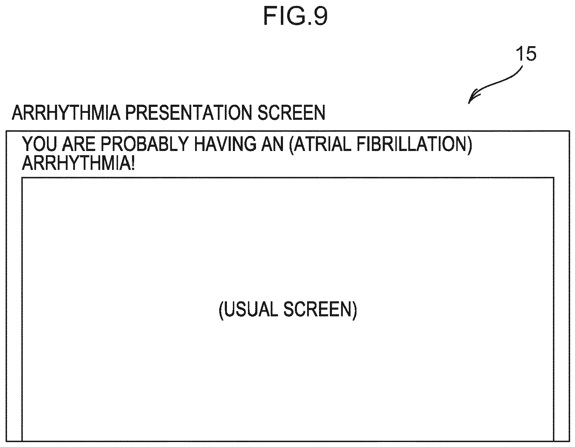

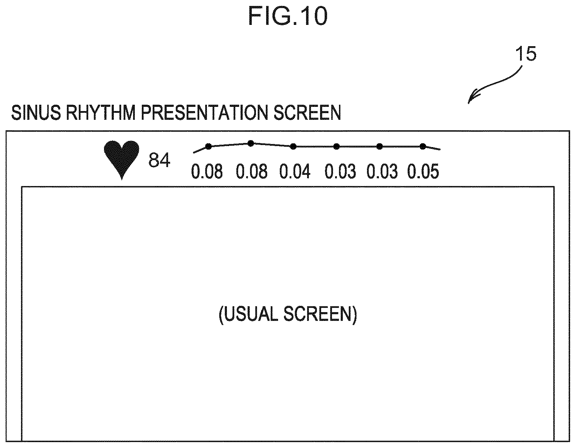

Now, operation of the arrhythmic state detection assisting device 10 according to the present exemplary embodiment is described with reference to Fig. 8 to Fig. 10. Fig. 8 is a flowchart showing an example of the arrhythmic state detection assisting processing according to the exemplary embodiment. Fig. 9 is an elevation view showing an example of composition of an arrhythmia presentation screen that is displayed at a time of execution of the arrhythmic state detection assisting processing according to the exemplary embodiment. Fig. 10 is an elevation view showing an example of composition of a sinus rhythm presentation screen that is displayed at a time of execution of the arrhythmic state detection assisting processing according to the exemplary embodiment.

The arrhythmic state detection assisting processing shown in Fig. 8 is implemented by the CPU 11 of the arrhythmic state detection assisting device 10 executing the arrhythmic state detection assisting program 13A. The arrhythmic state detection assisting processing shown in Fig. 8 is executed when a user performs entry of a command that starts execution of the arrhythmic state detection assisting program 13A via the entry unit 14. At this time, the user positions the arrhythmic state detection assisting device 10 at a position at which imaging of the user's face by the image capture unit 20 is possible.

In step 100 of Fig. 8, the CPU 11 performs control to start image capture of a color moving image by the image capture unit 20. In step 102, the CPU 11 acquires from the image capture unit 20 image information representing a single color image frame in the moving image obtained by the image capture.

In step 104, the CPU 11 extracts a value of the green luminance component from the acquired image information. In the present exemplary embodiment, the extraction of the green luminance component value is conducted as described below.

First, the CPU 11 detects parts at two portions, the forehead and left cheek, of the face of the user from the acquired image information. The present exemplary embodiment implements detection of the forehead and left cheek at this time by detecting regions of the face of the user with a conventionally known facial recognition technology and identifying, in skin-colored regions of the face, a partial region including an upper end portion as a forehead region and a partial region including a right end portion as a left cheek region. However, this mode is not limiting. Obviously a mode that uses an alternative conventionally known image recognition technology for detecting parts at the two portions of the forehead and left cheek is possible.

Then, for each of the parts in the acquired image information, the forehead and left cheek, the CPU 11 extracts values of the green luminance component from the image information of the detected regions of the forehead and left cheek, and calculates mean values of the green luminance component values for each of those parts. Considering the mean value that is larger among the calculated mean values of the respective parts to be less likely to be adversely affected by various kinds of noise, the CPU 11 employs the larger mean value as the green luminance component value.

Thus, in the present exemplary embodiment, the largest of the mean values of green luminance component values at plural portions (in the present exemplary embodiment, two portions, the forehead and the left cheek) is employed as the green luminance component, but this is not limiting. For example, a mode is possible in which a mean value of the mean values of green luminance component values of the plural parts is employed as the green luminance component.

In step 106, the CPU 11 memorizes the extracted value of the green luminance component into the green luminance component information database 13B together with an elapsed time from the start of image capture by the image capture unit 20. In step 108, the CPU 11 makes a determination as to whether a predetermined first interval (in the present exemplary embodiment, 3 minutes) has elapsed. When the result of this determination is affirmative, the CPU 11 proceeds to step 112, and when the result of the determination is negative, the CPU 11 proceeds to step 110. In step 110, the CPU 11 waits for a predetermined second interval (in the present exemplary embodiment, 0.05 s) to elapse, and then returns to step 102.

The green luminance component information database 13B, for example, as shown in Fig. 3, is sequentially built up by repeating the processing of step 102 to step 110 described above.

In step 112, the CPU 11 reads the green luminance component values corresponding to the most recent predetermined interval (3 minutes in the present exemplary embodiment) from the green luminance component information database 13B, and uses the green luminance component values that are read to create a pulse wave waveform (equivalent to the video pulse wave mentioned above).

In step 114, the CPU 11 uses the created pulse wave waveform to derive the above-described variance value V. In step 116, the CPU 11 makes a determination as to whether the derived variance value V is at least a threshold value TH (in the present exemplary embodiment, 0.13). When the result of this determination is affirmative, the CPU 11 considers the likelihood that an arrhythmia is occurring to be high and proceeds to step 118.

In step 118, the CPU 11 uses a pattern of the created pulse wave waveform (below referred to as the detected pulse wave pattern) to identify a type of arrhythmia that is likely to be occurring (below referred to as "the arrhythmia type"), as illustrated below.

First, the CPU 11 reads all the information (below referred to as the arrhythmia pattern information) from the arrhythmia pattern information database 13C. Then the CPU 11 identifies a pattern that most resembles the detected pulse wave pattern from the patterns represented by the read arrhythmia pattern information. In the present exemplary embodiment, a conventionally known pattern matching technology is used for identifying the pattern, but this is not limiting.

As the type of arrhythmia, the CPU 11 identifies a type of arrhythmia represented by the arrhythmia name corresponding to the identified pattern in the arrhythmia pattern information that most resembles the detected pulse wave pattern.

In step 120, the CPU 11 controls the display unit 15 so as to display an arrhythmia presentation screen with a predetermined composition, and subsequently proceeds to step 124.

Fig. 9 shows an example of the composition of the arrhythmia presentation screen according to the exemplary embodiment. As shown in Fig. 9, in the arrhythmia presentation screen according to the present exemplary embodiment, information indicating that the likelihood that an arrhythmia is occurring is high is displayed together with information representing the identified type of arrhythmia (in the example shown in Fig. 9, "atrial fibrillation"). Thus, by referring to the arrhythmia presentation screen, a user may understand that the likelihood they are having an atrial fibrillation is high.

Alternatively, when the result of the determination in step 116 is negative, the CPU 11 considers the likelihood that an arrhythmia is occurring to be low and proceeds to step 122.

In step 122, the CPU 11 controls the display unit 15 so as to display a sinus rhythm presentation screen with a predetermined composition, and subsequently proceeds to step 124.

Fig. 10 shows an example of composition of the sinus rhythm presentation screen according to the exemplary embodiment. As shown in Fig. 10, the sinus rhythm presentation screen according to the present exemplary embodiment displays a graph showing a pulse rate of the user at that moment and a graph showing recent estimates of the variance value V. Thus, by referring to the sinus rhythm presentation screen, the user may understand that the likelihood they are having an arrhythmia is low and the recent estimates of the variance value V.

In step 124, the CPU 11 makes a determination as to whether a predetermined end timing has been reached. When the result of this determination is negative, the CPU 11 returns to step 102, and when the result of the determination is affirmative, the CPU 11 proceeds to step 126. In the present exemplary embodiment, a timing at which an entry commanding the end of execution of the arrhythmic state detection assisting program 13A is carried out by the user via the entry unit 14 is employed as the end timing, but this is not limiting. For example, a mode in which an alternative timing is employed as the end timing is possible, such as a timing at which the face of the user has been removed from a field of image capture by the image capture unit 20 for at least a predetermined interval (for example, 10 seconds), or a pre-specified time or the like.

In step 126, the CPU 11 stops the image capture by the image capture unit 20 that was started by the processing of step 100, and subsequently ends the present arrhythmic state detection assisting processing.

As described above, according to the exemplary embodiment, the acquisition unit 11A, the derivation unit 11B and the estimation unit 11C are provided. The acquisition unit 11A acquires image information obtained by capturing an image of a predetermined part of a subject with an image capture device that captures a color moving image. The derivation unit 11B derives a variance value, for a predetermined interval, of periods of a periodic change in a green luminance component in the image information acquired by the acquisition unit 11A. When the variance value derived by the derivation unit 11B is greater, the estimation unit 11C estimates a higher likelihood that an arrhythmia is occurring in the subject. Thus, whether or not an arrhythmia is occurring may be more accurately estimated.

According to the exemplary embodiment, a high likelihood that an arrhythmia is occurring is estimated when the variance value is at least the predetermined threshold value. Thus, whether or not the likelihood that an arrhythmia is occurring is high may be estimated more easily than if this threshold value is not used.

According to the exemplary embodiment, the part is at the face of the subject. Thus, a state of arrhythmia may be estimated more easily then if the part is not at the face.

According to the exemplary embodiment, the part is parts at plural portions of the face. Thus, a state of arrhythmia may be estimated more accurately than if the part is not parts at plural portions.

According to the exemplary embodiment, when the part moves in the moving image represented by the acquired image information, movement of the part is tracked for deriving the variance value. Thus, a state of arrhythmia may be estimated more accurately than if the variance value is derived without tracking movement of the part.

According to the exemplary embodiment, when the subject has an arrhythmia, a chronological pattern of the green variance component is used to identify a type of the arrhythmia. Thus, convenience for the user may be further improved compared to an embodiment that does not identify a type of the arrhythmia.

In the exemplary embodiment described above, a case is described in which a state of occurrence of arrhythmia is presented by a display by the display unit, but this is not limiting. For example, a mode is possible in which a state of occurrence of arrhythmia is presented by a voice message by the sound output unit 19, and a mode is possible in which a state of occurrence of arrhythmia is presented by printing by an image forming device such as a printer or the like.

In the exemplary embodiment described above, a case is described in which the arrhythmic state detection assisting device of the present invention is structured by a device that is formed as a single structure (in the above exemplary embodiment, a smartphone), but this is not limiting. Modes are possible in which the arrhythmic state detection assisting device relating to the present invention is structured by a system using plural devices, for example, a server device such as a cloud server or the like and a terminal device. In this case, a mode may be illustrated that captures a color moving image of the user with the terminal device, transfers the color moving image to the server device, uses the received moving image at the server device to estimate a state of occurrence of arrhythmia, sends an estimation result to the terminal device, and presents the estimation result at the terminal device.

In the exemplary embodiment described above, a case is described in which the likelihood that a subject is having an arrhythmia is estimated to be high when the variance value V is equal to or greater than the threshold value TH, but this is not limiting. For example, it may be that the greater the variance value V, the higher the likelihood that an arrhythmia is occurring in a subject is estimated to be. A variant example in this case may be illustrated in which, in the processing of step 120 of the arrhythmic state detection assisting processing (see Fig. 8), the arrhythmia presentation screen displays information indicating that the greater the variance value, the more likely it is that an arrhythmia is occurring in the subj ect.

Various kinds of processor illustrated below may be used as hardware structures of the processing unit of the exemplary embodiment described above that executes the processing of, for example, the acquisition unit 11A, the derivation unit 11B, the estimation unit 11C and the identifying unit 11D. These various kinds of processor include, in addition to a CPU that is a general-purpose processor that functions as a processing unit executing software (a program) as described above, a PLD (programmable logic device) in which a circuit configuration can be modified after fabrication, such as an FPGA (field programmable gate array) or the like, a dedicated electronic circuit which is a processor with a circuit configuration that is specially designed to execute specific processing, such as an ASIC (application-specific integrated circuit) or the like, and so forth.

The processing unit may be structured by one of these various kinds of processors, and may be structured by a combination of two or more processors of the same or different kinds, for example, a combination of plural FPGAs, a combination of a CPU with an FPGA, or the like. The processing unit may also be structured by a single processor.

Examples in which the processing unit is structured by a single processor include: firstly, a mode in which the single processor is structured by a combination of one or more CPUs and software, of which client and server computers or the like are representative, and this processor functions as the processing unit; and secondly, a mode in which the functions of an entire system including the processing unit are realized by a single integrated circuit (IC), of which a system on chip (SoC) or the like is representative. Thus, the processing unit is structured using one or more of the above-mentioned various kinds of processor as a hardware structure.

As hardware structures of these various kinds of processor, in more concrete terms, electronic circuitry combining circuit elements such as semiconductor components and the like may be employed.

The disclosures of Japanese Patent Application No. 2020-216927 filed December 25, 2020 are incorporated into the present specification by reference in their entirety. All references, patent applications and technical specifications cited in the present specification are incorporated by reference into the present specification to the same extent as if the individual references, patent applications and technical specifications were specifically and individually recited as being incorporated by reference.

The exemplary embodiment described above may be a mode of a non-transitory storage medium memorizing a program. Examples of a non-transitory recording medium that can be expected include a CD-ROM (compact disc read-only memory), a magneto-optical disc, an HDD, a DVD-ROM (digital versatile disc read-only memory), a flash memory, a memory card and the like.

The following supplementary note is disclosed in relation to the exemplary embodiment described above.

- Supplementary note 1 -

A non-transitory storage medium stores a program executable by a computer to perform arrhythmic state detection assisting processing,
the arrhythmic state detection assisting processing including:

acquiring image information obtained by capturing an image of a predetermined part of a subject with an image capture device that captures a color moving image;
deriving a variance value, for a predetermined interval, of periods of a periodic change in a green luminance component in the acquired image information; and,
as the derived variance value becomes greater, estimating a higher likelihood that an arrhythmia is occurring in the subject.

**Claims**

1. An arrhythmic state detection assisting device comprising:

an acquisition unit that acquires image information obtained by capturing an image of a predetermined part of a subject with an image capture device that captures a color moving image;
a derivation unit that derives a variance value, for a predetermined interval, of periods of a periodic change in a green luminance component in the image information acquired by the acquisition unit; and
an estimation unit that, as the variance value derived by the derivation unit becomes greater, estimates a higher likelihood that an arrhythmia is occurring in the subject.

2. The arrhythmic state detection assisting device according to claim 1, wherein the estimation unit estimates a higher likelihood that an arrhythmia is occurring when the variance value is at least a predetermined threshold value.

3. The arrhythmic state detection assisting device according to claim 1 or claim 2, wherein the part is a face of the subject.

4. The arrhythmic state detection assisting device according to claim 3, wherein the part is parts at a plurality of portions of the face.

5. The arrhythmic state detection assisting device according to any one of claims 1 to 4, wherein, when the part moves in the moving image represented by the image information acquired by the acquisition unit, the derivation unit tracks movement of the part for deriving the variance value.

6. The arrhythmic state detection assisting device according to any one of claims 1 to 5, further comprising an identifying unit that, when the subject has an arrhythmia, identifies a type of the arrhythmia, the identifying unit using a chronological pattern of the green luminance component.

7. A program executable by a computer to perform processing comprising:

acquiring image information obtained by capturing an image of a predetermined part of a subject with an image capture device that captures a color moving image;
deriving a variance value, for a predetermined interval, of periods of a periodic change in a green luminance component in the acquired image information; and,
as the derived variance value becomes greater, estimating a higher likelihood that an arrhythmia is occurring in the subject.

# FIG.1

ARRHYTHMIC STATE DETECTION ASSISTING DEVICE — 10

11 — CPU

16 — R/W

17

12 — MEMORY

14 — ENTRY UNIT

15 — DISPLAY UNIT

19 — SOUND OUTPUT UNIT

20 — IMAGE CAPTURE UNIT

13 — STORAGE UNIT

13A — ARRHYTHMIC STATE DETECTION ASSISTING PROGRAM

13B — GREEN LUMINANCE COMPONENT INFORMATION DATABASE

13C — ARRHYTHMIA PATTERN INFORMATION DATABASE

18 — WIRELESS COMMUNICATION UNIT

B

FIG.2

# FIG.3

GREEN LUMINANCE COMPONENT
INFORMATION DATABASE

| ELAPSED TIME | GREEN LUMINANCE COMPONENT |
|---|---|
| 0.00 | 123 |
| 0.01 | 124 |
| ⋮ | ⋮ |

# FIG.4

ARRHYTHMIA PATTERN INFORMATION DATABASE

| ARRHYTHMIA NAME | PATTERN |
|---|---|
| ATRIAL FIBRILLATION | |
| PREMATURE ATRIAL CONTRACTION | |
| SINOATRIAL BLOCK | · · · |
| · · · | · · · |

FIG.5

# FIG.6

# FIG.7

$P_1$  $P_2$

sec

VIDEO
PULSE WAVES

PHOTOELECTRIC
PULSE WAVES

INSTANTANEOUS PULSE RATE (PULSES/MINUTE) = $60/P_1-P_2$

FIG.8

```
        ┌─────────────────────────────────┐
        │  ARRHYTHMIC STATE DETECTION     │
        │     ASSISTING PROCESSING        │
        └─────────────────────────────────┘
                         │
        ┌─────────────────────────────────┐
        │      START IMAGE CAPTURE         │──100
        └─────────────────────────────────┘
                         │
        ┌─────────────────────────────────┐
        │     ACQUIRE IMAGE INFORMATION    │──102
        └─────────────────────────────────┘
                         │
        ┌─────────────────────────────────┐
        │  EXTRACT GREEN LUMINANCE COMPONENT│──104
        └─────────────────────────────────┘
                         │
        ┌─────────────────────────────────┐
        │  MEMORIZE GREEN LUMINANCE COMPONENT│──106
        └─────────────────────────────────┘
                         │
              108    ◇ FIRST INTERVAL ELAPSED ?
           N ──      ◇
                      │ Y
       N ──◇ SECOND INTERVAL ELAPSED ?
              │ 110
              │ Y
        ┌─────────────────────────────────┐
        │    CREATE PULSE WAVE WAVEFORM    │──112
        └─────────────────────────────────┘
                         │
        ┌─────────────────────────────────┐
        │      DERIVE VARIANCE VALUE V     │──114
        └─────────────────────────────────┘
                         │
              116  ◇ V ≥ TH ?  ── N
                      │ Y
        ┌──────────────┐       ┌─────────────────────┐
        │  IDENTIFY    │──118  │     DISPLAY         │──122
        │ ARRHYTHMIA   │       │   SINUS RHYTHM      │
        │   TYPE       │       │ PRESENTATION SCREEN │
        └──────────────┘       └─────────────────────┘
                │ 120
        ┌──────────────┐
        │   DISPLAY    │
        │ ARRHYTHMIA   │
        │ PRESENTATION │
        │   SCREEN     │
        └──────────────┘
                         │
              124  ◇ END TIMING REACHED ? ── N
                      │ Y
        ┌─────────────────────────────────┐
        │      STOP IMAGE CAPTURE          │──126
        └─────────────────────────────────┘
                         │
                   ┌──────────┐
                   │   END    │
                   └──────────┘
```

$V \geq TH$

18

# FIG.9

15

ARRHYTHMIA PRESENTATION SCREEN

YOU ARE PROBABLY HAVING AN (ATRIAL FIBRILLATION)
ARRHYTHMIA!

(USUAL SCREEN)

# FIG.10

15

SINUS RHYTHM PRESENTATION SCREEN

♥ 84   0.08  0.08  0.04  0.03  0.03  0.05

(USUAL SCREEN)

## INTERNATIONAL SEARCH REPORT

| International application No. |
| --- |
| **PCT/JP2021/047384** |

**A.     CLASSIFICATION OF SUBJECT MATTER**

*A61B 10/00*(2006.01)i; *A61B 5/02*(2006.01)i
FI:    A61B5/02 310Z; A61B10/00 K

According to International Patent Classification (IPC) or to both national classification and IPC

**B.      FIELDS SEARCHED**

Minimum documentation searched (classification system followed by classification symbols)

A61B5/02-5/0295; A61B5/00; A61B10/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII)

**C.      DOCUMENTS CONSIDERED TO BE RELEVANT**

| Category* | Citation of document, with indication, where appropriate, of the relevant passages | Relevant to claim No. |
| --- | --- | --- |
| A | WO 2017/085896 A1 (FUJITSU LTD) 26 May 2017 (2017-05-26)<br>paragraphs [0010]-[0263], fig. 1-25 | 1-7 |
| A | JP 2016-159108 A (OMRON CORPORATION) 05 September 2016 (2016-09-05)<br>paragraphs [0013]-[0039], fig. 1-4 | 1-7 |
| A | US 2016/0317041 A1 (THE BOARD OF TRUSTEES OF THE UNIVERSITY OF ILLINOIS)<br>03 November 2016 (2016-11-03)<br>paragraphs [0049]-[0110], fig. 1-17 | 1-7 |

☐ Further documents are listed in the continuation of Box C.          ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **04 March 2022** | **15 March 2022** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

EP 4 268 730 A1

**INTERNATIONAL SEARCH REPORT**
Information on patent family members

International application No.

**PCT/JP2021/047384**

| Patent document cited in search report | | | Publication date (day/month/year) | Patent family member(s) | | | Publication date (day/month/year) |
|---|---|---|---|---|---|---|---|
| WO | 2017/085896 | A1 | 26 May 2017 | US paragraphs [0038]-[0322], fig. 1-25 EP | 2018/0256047 3378384 | A1 A1 | |
| JP | 2016-159108 | A | 05 September 2016 | US paragraphs [0015]-[0046], fig. 1-4 WO | 2017/0347898 2016/139871 | A1 A1 | |
| US | 2016/0317041 | A1 | 03 November 2016 | WO p. 10, line 20 to p. 28, line 3, fig. 1-17 CA | 2015/095760 2934659 | A1 A1 | |

Form PCT/ISA/210 (patent family annex) (January 2015)

22

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019058258 A
- JP 2019136352 A
- JP 2017085894 A
- US 10004410 B
- JP 2020216927 A